# EUROPÄISCHE PATENTANMELDUNG

(11) **EP 2 018 856 A2**
(43) Veröffentlichungstag der Anmeldung: **28.01.2009**
(21) Anmeldenummer: 08159988.8
(22) Anmeldetag: 09.07.2008
(51) Int. Cl.: A61K 31/26, A61K 31/17, A61K 31/715, A61K 31/728, A61K 33/40, A61K 45/06, A61P 17/02, A61P 39/00

(54) **Mehrteiliges Medizinprodukt zur Wundreinigung und -heilung**

(30) Priorität: 23.07.2007 DE 102007034639
(71) Anmelder: Antiseptica Chemisch-Pharmazeutische Produkte GmbH, 50259 Pulheim-Brauweiler (DE)
(72) Erfinder: Assadian, Ojan, 1180, Wien (AT); Bannert, Christian, 86199, Augsburg (DE); Weber, Ulrike, 17489, Greifswald (DE); Kramer, Axel, 17493, Greifswald (DE); Below, Harald, 17493, Greifswald (DE)
(74) Vertreter: Jönsson, Hans-Peter

(57) **Zusammenfassung**

Gegenstand der Erfindung ist ein mehrteiliges Medizinprodukt zur Reinigung von Wunden, mit dem zugleich die Wundheilung durch ein physiologisch vorkommendes System unterstützt werden soll.

## Beschreibung

Gegenstand der Erfindung ist ein mehrteiliges Medizinprodukt zur Reinigung von Wunden und Unterstützung der natürlichen Wundheilung durch ein physiologisch vorkommendes System.

DE 197 10 068 A1 betrifft Mittel zur Förderung der Mundhygiene und der Mundgesundheit, insbesondere zu Hemmung der Akkumulation bakterieller Plaque auf Zähnen, Zahnersatz und jeweils angrenzenden Oberflächen zur Prophylaxe sowie zur Therapie von Entzündungen und Erkrankungen der Mundschleimhaut, der Gingiva, des Parodontiums sowie zur Kariesprophylaxe. Diese Mittel dienen der Förderung der Mundhygiene und umfassen ionisch gebundene oder freie Thiocyanationen und Carbamid-Perhydrat.

DE 35 20 921 A1 beschreibt viskoelastische Zusammensetzungen auf der Basis von Wasser, die ein Gemisch von Hyaluronsäuren von hoher Molekularmasse und wasserlöslichen Polyethylenoxiden aufweisen. Gleichzeitig offenbart werden kosmetische Rezepturen, die solche Zusammensetzungen enthalten.

Demgegenüber stellt sich die Aufgabe der vorliegenden Erfindung darin, eine mehrteiliges Medizinprodukt zur Reinigung und Unterstützung der Heilung von Wunden des menschlichen oder tierischen Körpers zur Verfügung zu stellen, um die Wundheilung vor allem auf mechanischem Weg durch ein physiologisch vorkommendes System zu unterstützen. Die vorgenannte Aufgabe wird in einer ersten Ausführungsform durch ein mehrteiliges Medizinprodukt zur Reinigung und Unterstützung der Heilung von Wunden des menschlichen oder tierischen Körpers gelöst.

Das mehrteilige Medizinprodukt umfasst:
a) eine wässrige Reinigungslösung enthaltend
   aa) ionisch gebundene oder freie Thiocyanationen, insbesondere zur physiologische Reinigung durch Veränderung des Sol-Gel-Zustands von Eiweiß sowie durch Bildung von OSCN- und zur Förderung der Wundheilung und
   ab) wenigstens ein oder mehrere physiologisch verträgliche Zuckertenside, insbesondere zur Reinigungsverstärkung,
   ac) Hyaluronsäure und/oder Alkalimetallsalze der Hyaluronsäure, insbesondere zur Viskositätseinstellung und Wundheilung und
   ad) Puffersystem im pH-Bereich 3,0 bis 8,0, insbesondere zur physiologischen Reinigung durch Einstellung des OSCN-/HOSCN Verhältnisses,
   und
b) Carbamid-Perhydrat, insbesondere zur Reinigung und Verstärkung der physiologische Reinigung durch Bildung von OSCN-.

Diese werden in einem 2-Komponenten-System bereitgestellt, wobei die Komponenten a) und b) voneinander getrennt sind.

Das erfindungsgemäße Medizinprodukt verbindet die Reinigung von Wunden mit nachfolgender Unterstützung der Wundheilung. Bedingt durch die Unterstützung der Wundheilung durch ein physiologisch vorkommendes System ist das erfindungsgemäße Medizinprodukt vor allem für die Versorgung von verschmutzten, aber auch von chronischen Wunden geeignet.

Die für die Wirksamkeit notwendige Enzyme stehen im Applikationsbereich physiologisch zur Verfügung.

Eine zusätzliche Verbesserung der Wundreinigung wird insbesondere durch die Bildung von OSCN- durch das mehrteilige Medizinprodukt in Verbindung mit körpereigenen Enzymen erreicht. Dazu kann durch die Einstellung des pH-Wertes das HOSCN/OSCN- Verhältnis optimiert und an das Applikationsareal angepasst werden. Ein pH-Wert von 8 verschiebt das Gleichgewicht vollständig auf die Seite von OSCN-, das hier stabil ist, während bei einem pH-Wert von 3,0 ausschließlich HOSCN stabil vorliegt. HOSCN unterscheidet sich gegenüber dem geladenen OSCN- durch eine höhere Lipophilie. Der pkₐ-Wert des System, HOSCN/OSCN- beträgt 5,3 das heißt bei einen pH-Wert von 5,3 liegen beide Komponenten in gleichen Verhältnissen vor, andere pH-Werte zwischen 3 und 8 stellen beliebige Verhältnisse ein.

Mit diesem System wird das Lecken von Wunden aus dem Tierreich nachgeahmt. Thiocyanat fördert die Proliferation und Radikalentgiftung. Auf die Wunde aufgetragen, bilden sich mit aus dem Carbamid-Perhydrat und körpereigenen Peroxidasen sekundär OSCN- und O₂SCN⁻ als natürliches Wundreinigungsmittel.

Besonders bevorzugt im Sinne der vorliegenden Erfindung liegen die Thiocyanationen in der wässrigen Reinigungslösung in Form von Alkalimetallsalzen einschließlich der Ammoniumsalze und ihrer Derivate vor. Hierbei handelt es sich somit um dissoziationsfähige Thiocyanate im Gegensatz zu organischen, kovalent gebundenen Thiocyanaten. Besonders bevorzugt im Sinne der vorliegenden Erfindung sind die Alkalimetallsalze der Thiocyanwasserstoffsäure ausgewählt aus Natrium-, Kalium- und/oder Ammoniumthiocyanat.

Im Sinne der vorliegenden Erfindung enthalten die Mittel besonders bevorzugt 0,01 bis 30 g/kg Thiocyanationen. Diese und alle nachfolgenden Mengenangaben beziehen sich auf die Gesamtmenge aller Bestandteile der Komponenten a) oder b).
Die erfindungsgemäßen Reinigungslösungen des mehrteiligen Medizinproduktes umfassen Hyaluronsäuren, Alkalimetallsalze der Hyaluronsäuren oder deren Gemische in einer Menge von 0,01 bis 2 Ges.-%, insbesondere mit Hyaluronsäureresten einer mittleren Molmasse im Bereich von 1.000 bis 5.000 d.

Hyaluronsäure ist bekanntermaßen ein hochmolekulares, hochviskose Lösungen bildendes Mucopolysaccharid, das aus etwa gleichmolaren Mengen Glucuronsäure und N-Acetylglucuranamin bzw. -glucosamin besteht. Molekularmassen umfassen üblicherweise 5 x 10⁴ bis 8 x 10⁶ Dalton. Hyaluronsäure mit einer Molmasse in Bereich von 1000 bis 5000 d dringt leicht in die Haut ein und verbessert das Feuchthaltevermögen des Stratum corneum deutlich. Je größer die Molmasse der Hyaluronsäure, umso besser ausgeprägt ist die Fähigkeit, dass Feuchthaltevermögen des Stratum corneum zu verbessern.

Hyaluronsäure ist wichtiger Bestandteil des Bindegewebes mit Förderung der Zellproliferation und Zellwanderung. Hyaluronsäure bindet sich an spezifische Rezeptoren auf den Oberflächen der verschiedensten Zellen. Dadurch ist sie in der Lage, akute und chronische Entzündungsreaktionen zu modulieren. Die Wundheilung, die ein komplexes Aufeinanderfolgen streng regulierter Reaktionen wie Entzündung, Bildung von Granulationsgeweben, Reepithelialisierung und Remodelierung darstellt, wird an verschiedenen Stadien dieser Prozesse durch Hyaluronsäure moduliert. Hyaluronsäure hat die Fähigkeit, relativ zu ihrer Masse sehr große Mengen an Wasser zu binden (bis zu sechs Liter Wasser pro Gramm). So besteht der Glaskörper des Auges zu 98 Gew.-% aus Wasser, das an nur 2 Gew.-% Hyaluronsäure gebunden ist. Daher ist es ein effizientes Tränenersatzmittel und Zusatz zu Kontaktlinsenreinigern.

Neben Thiocyanationen, einem Puffersystem und Hyaluronsäure enthält die wässrige Reinigungslösung des erfindungsgemäßen mehrteiligen Medizinprodukts Zuckertenside, die insbesondere ausgewählt sind aus der Gruppe der Alkylpolyglycoside, Glyceride, Saccharose- (Sucrose-) Ester, Sorbitanester, Glucoseester oder deren Gemische. Die Alkylpolyglycoside (APG) sind im Rahmen des erfindungsgemäßen Medizinproduktes besonders bevorzugte Zuckertenside und genügen vorzugsweise der allgemeinen Formel

R¹O(AO)ₐ[G]ₓ

in der R¹ für einen linearen oder verzweigten, gesättigten oder ungesättigten Alkylrest oder Alkenylrest mit 6 bis 22 Kohlenstoffatomen, [G] für einen glycosidisch verknüpften Zuckerrest und x für eine Zahl von 1 bis 10 sowie AO für eine Alkylenoxid-Gruppe, zum Beispiel eine Ethylenoxid- oder Propylenoxid-Gruppe, und a für den mittleren Alkoxylierunggrad von 0 bis 20 stehen. APGs sind nichtionische Tenside und stellen bekannte Stoffe dar, die nach einschlägigen Verfahren der präparativen organischen Chemie erhalten werden können. Die Indexzahl x gibt den Oligomerisierungsgrad (TP-Grad) an, das heißt die Verteilung von Mono- und Oligoglycosiden und steht für eine Zahl zwischen 1 und 10.

Vorzugsweise leitet sich der Alkyl- bzw. Alkenylrest R¹ von Laurylalkohol, Myristylalkohol, Cetylalkohol, Palmoleylalkohol, Stearylalkohol, Isostearylalkohol oder Oleylalkohol ab. Besonders bevorzugter glycosidisch verknüpfter Zuckerrest ist der Glucoserest.

Besonders bevorzugt umfasst die erfindungsgemäße Reinigungslösung 0,5 bis 5 Gew.-% Zuckertenside.

Die erfindungsgemäße Reinigungslösung Komponente b) enthält Carbamid-Perhydrat, insbesondere in wässriger Lösung. Carbamid-Perhydrat ist ein Harnstoff-Wasserstoffperoxid-Addukt im Verhältnis 1:1 und stellt somit eine Kombination von zwei physiologischen Wirkstoffen dar. Carbamid-Perhydrat wirkt als Wasserstoffperoxid-Lieferant für die OSCN⁻-Bildung und hat anstelle von enzymatisch gebildetem Wasserstoffperoxid die folgenden Vorteile:
- Wasserstoffperoxid-Bereitstellung durch direkte Abspaltung anstelle eines Umweges über störanfällige biochemische Bildung,
- Verbesserung der sekundären Wirkstoffpenetration durch den aus Carbamid-Perhydrat abgespaltenen Harnstoff (Wohlrab W.: Bedeutung von Harnstoff in der externen Therapie; Hautarzt 40, Suppl. 9: 1981).

Besonders bevorzugt im Sinne der vorliegenden Erfindung enthält die wässrige Reinigungslösung 0,01 bis 30 g/kg Carbamid-Perhydrat. Reinigung kann beispielsweise durch Einsatz von 1 g/kg Carbamid-Perhydrat (entspricht 0,4 g Wasserstoffperoxid, das mit Myelo- und Eosinophilenperoxidase zu OSCN- und O₂SCN⁻ umgesetzt wird) erreicht werden.

Das Mengenverhältnis der Komponenten a) zu Komponente b) ist weniger kritisch. Erfindungsgemäß ist es jedoch bevorzugt, wenn das Stoffmengenverhältnis von Carbamid-Perhydrat zu Thiocyanat 1:1 1 ist. Beim Überschreiten der Bereiche kann es zu einer direkten antiseptischen Wirkung von Wasserstoffperoxid oder zu einer Enzyminhibition des körpereigenen LPO-Systems kommen. Beim Unterschreiten der Bereiche wird die erwünschte Reinigungswirkung nicht erreicht.

Wunden im Sinne der vorliegenden Erfindung umfassen beispielsweise akute und chronische Wunden, Verbrennungswunden, kontaminierte traumatische Verletzungen.

### Ausführungsbeispiele:

- Gegenstand der Erfindung ist ein mehrteiliges Medizinprodukt zur Reinigung von Wunden und Unterstützung der natürlichen Wundheilung durch ein physiologisch vorkommendes System.
- getrennte Applikation der Komponenten der Reinigungslösungen aus einem Zweikomponenten-System als personenbezogenes Einmalprodukt mit Zusammenführung der Komponenten bei der Applikation
- Applikation der Reinigungslösungen aus einer Zweikomponenten-Tube als personenbezogenes Einmalprodukt mit einem mechanisch anwendbaren Tubenaufsatz (z.B. Bürste) zur Reinigungsverstärkung.

## Patentansprüche

1. Mehrteiliges Medizinprodukt umfassend:
a) eine wässrige Reinigungslösung enthaltend aa) ionisch gebundene oder freie Thiocyanationen,
ab) wenigstens ein oder mehrere physiologisch verträgliche Zuckertenside,
ac) Hyaluronsäure und/oder Alkalimetallsalze der Hyaluronsäure und
ad) Puffersystem im pH-Bereich 3,0 bis 8,0 und
b) Carbamid-Perhydrat.

2. Medizinprodukt nach Anspruch 1, **dadurch gekennzeichnet, dass** die Thiocyanationen in Form gelösten Alkalimetallsalzen, einschließlich der Ammoniumsalze vorliegen.

3. Medizinprodukt nach Anspruch 2, **dadurch gekennzeichnet, dass** die Alkalimetallsalze der Thiocyanationen ausgewählt sind aus Natrium-, Kalium- und/oder Ammoniumthiocyanat.

4. Medizinprodukt nach einem der Ansprüche 1 bis 3, **dadurch gekennzeichnet, dass** die Reinigungslösung a) Thiocyanationen in einer Menge von 0,01 bis 30 g/kg enthält.

5. Medizinprodukt nach einem der Ansprüche 1 bis 4, **dadurch gekennzeichnet, dass** die Reinigungslösung a) und/oder die Lösung b) Peroxidasen enthält

6. Medizinprodukt nach einem der Ansprüche 1 bis 4, **dadurch gekennzeichnet, dass** die Reinigungslösung a) und/oder die Lösung b) wasserstoffperoxidbildende Enzyme enthält.

7. Medizinprodukt nach einem der Ansprüche 1 bis 6, **dadurch gekennzeichnet, dass** die Zuckertenside ausgewählt sind aus der Gruppe der Alkylpolyglycoside, Glyceride, Saccharose-(Sucrose-)Ester, Sorbitanester, Glucoseester und deren Gemische.

8. Medizinprodukt nach einem der Ansprüche 1 bis 7, **dadurch gekennzeichnet, dass** die Lösung b) Carbamid-Perhydrat in einer Menge von 0,01 bis 30 g/kg enthält.

9. Verwendung eines Medizinproduktes nach einem der Ansprüche 1 bis 8 zur Reinigung und Dekontamination sowie sekundärer Desinfektion von Wunden des menschlichen oder tierischen Körpers.

10. Verwendung nach Anspruch 9 oder 10, **dadurch gekennzeichnet, dass** man in einem ersten Schritt die Reinigungslösung a) aufbringt und in einem zweiten Schritt Wasserstoffperoxid oder einen Wasserstoffperoxid-Abspalter aufbringt.
